# EUROPEAN PATENT SPECIFICATION

(11) **EP 3 037 812 B1**
(45) Date of publication and mention of the grant of the patent: **01.05.2024**
(21) Application number: 15201398.3
(22) Date of filing: 18.12.2015
(51) Int. Cl.: C12Q 1/54, C12Q 1/60, C12Q 1/26, C12Q 1/00, G01N 27/327

(54) **ENZYME ELECTRODE**
ENZYMELEKTRODE
ÉLECTRODE À ENZYME

(30) Priority: 24.12.2014 JP 2014261203
(43) Date of publication of application: 29.06.2016
(73) Proprietor: ARKRAY, Inc., Minami-ku Kyoto-shi, Kyoto 601-8045 (JP)
(72) Inventor: KANEDA, Hisashi, Kyoto-shi, Kyoto 602-0008 (JP)
(74) Representative: Dehns

(56) References cited:
- EP-A1- 1 679 508
- EP-A1- 2 573 190
- WO-A1-2005/066356
- WO-A1-2014/002998
- WO-A1-2014/002999
- WO-A2-01/46457
- US-A1- 2009 321 277
- US-A1- 2010 160 755

## Description

### TECHNICAL FIELD

The present invention relates to an enzyme electrode for measuring a charge transfer limiting current.

### BACKGROUND ART

An enzyme electrode is known which comprises an electrode as a base material, and a detection layer in which molecules of an enzyme and electrically conductive particles are immobilized on the surface of the electrode using a crosslinking agent or a binder. The enzyme electrode has a structure in which electrons generated by an enzyme reaction are transferred. Specifically, JP 2014-006154 A (Patent Document 1) discloses an enzyme electrode in which the detection layer thereof contains an enzyme, electrically conductive particles and a crosslinking agent. Further, JP 2014-006155 A (Patent Document 2) discloses an enzyme electrode in which the detection layer thereof contains an enzyme, electrically conductive particles, and an electrically conductive polymer.

US 2009/321277 discloses a cross-linked polymer matrix and its use as a bioelectrocatalyst on a non-corroding electrode. EP 2573190 discloses a lactate sensor which includes an insulating substrate, an electrode system including at least a working electrode and a counter electrode provided on the substrate, and a reagent layer provided on the electrode system. EP 1679508 discloses a biosensor and method for preparation thereof. US 2010/0160755 discloses an electrochemical sensor for measuring an analyte in a subject. WO 2014/002998 and EP 2866025 disclose an enzyme electrode including an electrode and a detection layer. WO 2014/002999 and EP 2866024 disclose an enzyme electrode which includes an electrode and a detection layer that is in contact with the electrode and includes an oxidoreductase, a water-soluble conductive polymer and conductive particles. WO 01/46457 discloses an amperometric biosensor test strip. WO 2005/066356 discloses a biosensor and method for its manufacture.

### SUMMARY OF THE INVENTION

An enzyme electrode is sometimes used for measuring the concentration of a target substance contained in a trace amount in a sample. In order to measure the concentration of the trace amount of the target substance, the sensitivity of the measurement needs to be improved. However, the enzyme electrodes disclosed in Patent Document 1 and Patent Document 2 still have room for improvement in the measurement sensitivity.

Namely, one aspect of the present invention is to provide an enzyme electrode for measuring the concentration of a target substance quantitatively and with a high sensitivity.

In order to achieve the above mentioned object, one aspect of the present invention adopts the following constitution.

Specifically, one aspect of the present invention relates to:
an enzyme electrode comprising:
an electrode; and
a detection layer which is in contact with the electrode and comprises an enzyme, a crosslinking agent, an electrically conductive polymer, and electrically conductive particles;
wherein electrons are transferred between the enzyme in the detection layer and the electrode and wherein the detection layer does not contain an electron transfer mediator being an oxidation-reduction substance,
characterized in that the crosslinking agent is an oxazoline group-containing compound, and the detection layer further comprises a sugar which is trehalose or raffinose.

The enzyme is preferably an oxidoreductase.

Another aspect of the present invention relates to a biosensor comprising the above mentioned enzyme electrode.

Further, another aspect of the present invention relates to:
a measuring apparatus comprising:
the above mentioned biosensor;
a control section configured to control the application of voltage to the biosensor;
a detection section configured to detect a charge transfer limiting current based on the transfer to the electrode of electrons derived from a substance to be measured, wherein the charge transfer limiting current is generated by the application of voltage to the biosensor;
an arithmetic section configured to calculate the concentration of the substance based on the value of the charge transfer limiting current; and
an output section configured to output the calculated concentration of the substance.

In addition, another aspect of the present invention relates to a method for producing an enzyme electrode comprising forming a detection layer by applying a mixed solution on an electrode, the mixed solution comprising an enzyme, a crosslinking agent which is an oxazoline group-containing compound, an electrically conductive polymer, electrically conductive particles, and a sugar which is trehalose or raffinose; wherein electrons are transferable between the enzyme and the electrode in the enzyme electrode and wherein the detection layer does not contain an electron transfer mediator which is an oxidation-reduction substance.

According to the present invention, an enzyme electrode having an improved measurement sensitivity and qualitative properties can be provided by incorporating a cross linking agent which is an oxazoline group-containing compound and a sugar which is trehalose or raffinose in the detection layer of the enzyme electrode.

### BRIEF DESCRIPTION OF THE DRAWINGS

FIG. 1 is a schematic diagram illustrating the structure of an enzyme electrode according to one embodiment of the present invention.
FIG. 2 is a schematic diagram illustrating one embodiment of a measuring apparatus according to the present invention.
FIG. 3 is a flow chart illustrating one embodiment of a measurement program using the measuring apparatus according to the present invention.
FIG. 4 is a graph of the current ratio (S/B), with (S) or without (B) glucose, showing the case where the enzyme electrode comprising a detection layer which contains no sugar is used (Comparative Example 1); and the case where the enzyme electrode comprising a detection layer which contains sucrose is used (Example 1).
FIG. 5 is a graph of the current ratio (S/B), with (S) or without (B) glucose, showing the case where enzyme electrodes each comprising a detection layer containing one of various types of sugar are used.

### EMBODIMENTS FOR CARRYING OUT THE INVENTION

The enzyme electrode as one embodiment of the present invention will now be described with reference to the drawings. The embodiment to be described below is provided only for illustration purposes, and the present invention is not limited to the constitution of the following embodiment.

### (Structure of enzyme electrode)

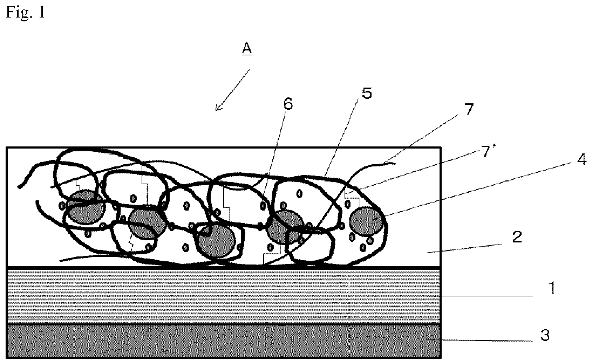

FIG. 1 is a schematic diagram of the enzyme electrode according to one embodiment of the present invention. In FIG. 1, an enzyme electrode A comprises an electrode 1; and a detection layer 2 formed on the surface of the electrode 1 (the upper surface, in FIG. 1).

### (Electrode)

The electrode 1 is made of a metallic material such as gold (Au), platinum (Pt), silver (Ag) or palladium (Pd); or a carbon material such as carbon. The electrode 1 is formed, for example, on an insulating base plate 3 as shown in FIG. 1. The insulating base plate 3 is made of an insulating material, and examples thereof include various types of resins (plastics), such as thermoplastic resins, for example, polyetherimide (PEI), polyethylene terephthalate (PET) and polyethylene (PE), polyimide resins, and epoxy resins; glasses; ceramics; papers; and the like. Any known material can be used as an electrode material for forming the electrode 1 and a material for forming the insulating base plate 3. The size and the thickness of the electrode 1 and the insulating base plate 3 can be determined as appropriate. Hereinafter, the combination of the insulating base plate 3 and the electrode 1 may be referred to as a "base material".

### (Detection layer)

The detection layer 2 is in contact with the electrode 1, and comprises an enzyme 4, an electrically conductive polymer 5, a sugar 6, electrically conductive particles and a crosslinking agent 7, but does not contain an electron transfer mediator which is an oxidation-reduction substance.

The enzyme electrode according to the present invention is used to measure a charge transfer limiting current based on the transfer to the electrode of electrons derived from the substance to be measured. The charge transfer limiting current is the current which is generated when the electrons are transferred from the enzyme to the electrode due to the reaction between the enzyme and the substance to be measured. Further, the charge transfer limiting current is a steady-state current which does not depend on time, and preferably, a steady-state current observed after the generation of a transient current due to the charging of an electric double layer.

In order to measure the charge transfer limiting current, a "direct electron transfer-type enzyme electrode" is used as a working electrode. The "direct electron transfer-type enzyme electrode" as used herein refers to a type of enzyme electrode in which electrons are transferred between the enzyme and the electrode in such a way that electrons generated by an enzyme reaction in a reagent layer are directly transferred to the electrode (including the case in which the transfer of electrons is mediated by an electrically conductive polymer) without the involvement of an oxidation-reduction substance as an electron transfer mediator.

As shown in FIG. 1, in the detection layer 2, the molecules of the enzyme 4 are crosslinked by the crosslinking agent 7, and further intertwined by the electrically conductive polymer 5 to exhibit a complex structure. The electrons generated by the enzyme reaction can be transferred to the electrode 1, directly or mediated by the electrically conductive polymer 5 which has an electrical conductivity. In other words, in the enzyme electrode A according to the embodiment of the invention, electrons are transferred between the enzyme 4 and the electrode 1 due to the direct electron transfer in the detection layer 2.

The limiting distance within which the direct electron transfer can occur in a physiological reaction system is reported to be from 10 to 20 Å. In the electron transfer in an electrochemical reaction system comprising an electrode and an enzyme, as well, the detection of the electron transfer on the electrode will be difficult if the distance between the electrode and the enzyme is longer than the above mentioned limiting distance, unless it involves the transfer (for example, transfer by diffusion) of a mediator. Therefore, in the detection layer 2, the active sites of the enzyme 4 (the sites at which electrons are generated due to the enzyme reaction) and the electrically conductive sites of the electrically conductive polymer 5 are located within a distance suitable for electron transfer, in other words, the electrically conductive sites and the active sites are located close enough so that electrons are transferred therebetween in a suitable manner.

### (Enzyme)

Examples of the enzyme 4 include oxidoreductases. Examples of the oxidoreductase include: glucose oxidase (GOD), galactose oxidase, bilirubin oxidase, pyruvic acid oxidase, D- or L-amino acid oxidase, amine oxidase, cholesterol oxidase, choline oxidase, xanthine oxidase, sarcosine oxidase, L-lactic acid oxidase, ascorbic acid oxidase, cytochrome oxidase, alcohol dehydrogenase, glutamate dehydrogenase, cholesterol dehydrogenase, aldehyde dehydrogenase, glucose dehydrogenase (GDH), fructose dehydrogenase, sorbitol dehydrogenase, lactate dehydrogenase, malate dehydrogenase, glycerol dehydrogenase, 17β hydroxysteroid dehydrogenase, estradiol 17β dehydrogenase, amino acid dehydrogenase, glyceraldehyde 3-phosphoric acid dehydrogenase, 3-hydroxysteroid dehydrogenase, diaphorase, cytochrome oxidoreductase, catalase, peroxidase, glutathione reductase, and the like. Among others, the enzyme 4 is preferably a saccharide oxidoreductase. Examples of the saccharide oxidoreductase include: glucose oxidase (GOD), galactose oxidase, glucose dehydrogenase (GDH), fructose dehydrogenase, and sorbitol dehydrogenase.

Further, the oxidoreductase can contain at least one of pyrroloquinoline quinone (PQQ) and flavin adenine dinucleotide (FAD), as a catalytic subunit and a catalytic domain. Examples of the oxidoreductase containing PQQ include PQQ glucose dehydrogenase (PQQGDH). Examples of the oxidoreductase containing FAD include cytochrome glucose dehydrogenase (Cy-GDH) and glucose oxidase (GOD), having an α-subunit containing FAD. In addition, the oxidoreductase can contain an electron transfer subunit or an electron transfer domain. Examples of the electron transfer subunit include a subunit containing heme which has the function of giving and receiving electrons. Examples of an oxidoreductase having a subunit containing heme include those containing cytochrome. For example, a fusion protein of glucose dehydrogenase or PQQGDH with cytochrome can be used. Further, examples of an enzyme containing an electron transfer domain include cholesterol oxidase and quinoheme ethanol dehydrogenase (QHEDH (PQQ Ethanol dh)). As the electron transfer domain, it is preferred to use a domain containing cytochrome containing heme which has the function of giving and receiving electrons. Examples thereof include "QHGDH" (fusion enzyme; GDH with heme domain of QHGDH)), sorbitol dehydrogenase (Sorbitol DH), D-fructose dehydrogenase (Fructose DH), Glucose-3-Dehydrogenase derived from *Agrobacterium tumefasience* (G3DH from *Agrobacterium tumefasience*), and cellobiose dehydrogenase. A fusion protein of PQQGDH with cytochrome, which is an example of the above mentioned subunit containing cytochrome, and a cytochrome domain of PQQGDH, which is an example of the domain containing cytochrome, are disclosed, for example, in WO 2005/030807. Further, as the oxidoreductase, it is preferred to use an oligomeric enzyme comprising at least a catalytic subunit and a subunit containing cytochrome containing heme which has a function as an electron acceptor.

### (Electrically conductive polymer)

Examples of the electrically conductive polymer 5, include: polypyrrole, polyaniline, polystyrene sulfonate, polythiophene, polyisothianaphthene, polyethylenedioxythiophene(poly(3,4-ethylenedioxythiophene)poly(styrene sulfonate)), and combinations thereof. Examples of the commercially available products thereof, specifically, examples of the commercially available product of polypyrrole include: "SSPY" (ethyl 3-methyl-4-pyrrolecarboxylate) (manufactured by KAKEN INDUSTRY Co., Ltd.). Examples of the commercially available product of polyaniline include "AquaPASS 01-x" (manufactured by TA Chemical Co., Ltd.), and the like. Examples of the commercially available product of polystyrene sulfonate include "Poly-NaSS" (manufactured by TOSOH ORGANIC CHEMICAL CO., LTD.). Examples of the commercially available product of polythiophene include "Espacer 100" (manufactured by TA Chemical Co., Ltd.). Examples of the commercially available product of polyisothianaphthene include "Espacer 300" (manufactured by TA Chemical Co., Ltd.)). Examples of the commercially available product of polyethylenedioxythiophene(poly(3,4-ethylenedioxythiophene)poly(styrene sulfonate)) include "PEDOT-PSS" (manufactured by Polysciences Inc.).

Further, as the electrically conductive polymer 5, an electrically conductive polymer having various attributes (for example, water solubility) can be used. It is preferred that the electrically conductive polymer 5 contain a hydroxyl group or a sulfo group as a functional group.

### (Sugar)

The sugar 6 is a sugar which does not serve as a substrate for the enzyme 4. The sugar 6 is trehalose or raffinose.

### (Crosslinking agent)

The crosslinking agent 7 is an oxazoline group-containing compound. Examples of oxazoline group-containing compounds include oxazoline compounds such as : 2,2'-bis-(2-oxazoline), 2,2'-methylene-bis-(2-oxazoline), 2,2'-ethylene-bis-(2-oxazoline), 2,2'-trimethylene-bis-(2-oxazoline), 2,2'-tetramethylene-bis-(2-oxazoline), 2,2'-hexamethylene-bis-(2-oxazoline), 2,2'-octamethylene-bis-(2-oxazoline), 2,2'-ethylene-bis-(4,4'-dimethyl-2-oxazoline), 2,2'-p-phenylene-bis-(2-oxazoline), 2,2'-m-phenylene-bis-(2-oxazoline), 2,2'-m-phenylene-bis-(4,4'-dimethyl-2-oxazoline), bis-(2-oxazolinylcyclohexane)sulfide, and bis-(2-oxazolinylnorbornane)sulfide. Further, examples of addition polymerizable oxazoline compounds include: 2-vinyl-2-oxazoline, 2-vinyl-4-methyl-2-oxazoline, 2-vinyl-5-methyl-2-oxazoline, 2-isopropenyl-2-oxazoline, 2-isopropenyl-4-methyl-2-oxazoline, 2-isopropenyl-5-ethyl-2-oxazoline, and the like. The compounds obtained by polymerization or copolymerization of one or more of these compounds can also be used. The oxazoline group-containing compounds are commercially available under the names of: Epocros WS-500, Epocros WS-700, Epocros K-1010E, Epocros K-1020E, Epocros K-1030E, Epocros K-2010E, Epocros K-2020E, Epocros K-2030E, Epocros RPS-1005 and Epocros RAS-1005 (all of the above are manufactured by NIPPON SHOKUBAI Co., Ltd.); and NK linker FX (manufactured by SHIN-NAKAMURA CHEMICAL Co., Ltd.). The type of the oxazoline group containing crosslinking agent is not limited to the above mentioned compounds and commercially available products. The form of the oxazoline group containing crosslinking agent is not limited, either. The oxazoline group containing crosslinking agent may be in the form of a monomer, polymer or the like.

### (Electrically conductive particles)

The detection layer further includes electrically conductive particles. As the electrically conductive particles, particles of a metal such as gold, platinum, silver or palladium; or a higher-order structure made of a carbon material can be used. The higher-order structure can contain, for example, one or more types of fine particles (carbon fine particles) selected from particles of electrically conductive carbon black, Ketjenblack (registered trademark), carbon nanotubes (CNT) and fullerene.

Further, the surface of the detection layer 2 may be covered with an outer-layer film made of cellulose acetate and the like. Examples of raw materials for the outer-layer film, in addition to cellulose acetate, include: polyurethane, polycarbonate, polymethyl methacrylate, butyl methacrylate, polypropylene, polyether ether ketone, and the like.

### (Method for preparing enzyme electrode)

The above mentioned enzyme electrode A is prepared, for example, as follows. Specifically, a metal layer which functions as the electrode 1 is formed on one surface of the insulating base plate 3. For example, a metal layer having a desired thickness (for example, about 30 nm) is formed by depositing a metallic material, by physical vapor deposition (PVD, for example, sputtering) or chemical vapor deposition (CVD), on one surface of the insulating base plate 3 in the form of a film having a predetermined thickness (for example, about 100 µm). It is also possible to form an electrode layer made of a carbon material, instead of the metal layer. Next, the detection layer 2 is formed on the electrode 1. Specifically, a solution (reagent) containing the enzyme 4, electrically conductive polymer 5, the sugar 6, the oxazoline group containing crosslinking agent 7, and the electrically conductive particles is prepared. The concentration of the sugar in the solution (reagent) is preferably from 0.1 to 2% by weight, and more preferably 0.2 to 2% by weight. The solution (reagent) is dropped on the surface of the electrode 1. Then the solution (reagent) is allowed to dry and solidify on the electrode 1, to obtain the enzyme electrode A in which the detection layer 2 is formed on the electrode 1.

By using the enzyme electrode according to the present invention, the concentration of the substance to be tested contained in a sample can be measured based on the charge transfer limiting current. The substance to be measured is not particularly limited as long as it can be measured by the measuring method using the enzyme electrode of the present invention. However, the substance to be measured is preferably a substance derived from a living body, which can serve as an index of a disease and/or health status, and examples thereof include glucose, cholesterol, and the like. The sample is not particularly limited as long as it contains the substance to be measured. However, a biological sample, such as blood or urine is preferred.

### (Biosensor)

The enzyme electrode according to the present invention can be used as a biosensor, such as a glucose sensor. The biosensor includes, along with the enzyme electrode of the present invention, an electrode which serves as a counter electrode. As the counter electrode, it is possible to use any electrode which can be generally used as the counter electrode in a biosensor. Examples thereof include: a carbon electrode prepared in the form of a film by screen printing; a metal electrode prepared in the form of a film by physical vapor deposition (PVD, for example, sputtering) or chemical vapor deposition (CVD); and a silver/silver chloride electrode prepared in the form of a film by screen printing. It is also possible to employ a 3-electrode system in which a silver/silver chloride electrode or a carbon electrode prepared in the form of a film by screen printing, or a metal electrode prepared in the form of a film by physical vapor deposition (PVD, for example, sputtering) or chemical vapor deposition (CVD), is used as a reference electrode.

### (Apparatus)

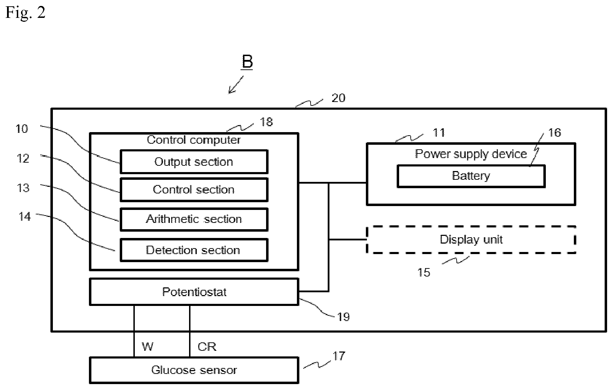

Next, the measuring apparatus according to the present invention will be described with reference to the drawings. Although a glucose measuring apparatus which includes a glucose sensor as the biosensor is illustrated in this embodiment, the measuring apparatus according to the present invention is not limited to the following embodiments. FIG. 2 shows an example of the configuration of main electronic components included in a measuring apparatus B. A control computer 18, a potentiostat 19 and a power supply device 11 are provided on a base plate 20 housed in a housing. The control computer 18 includes, as hardware, a processor such as CPU (central processing unit); a recording medium such as a memory (RAM (Random Access Memory) or ROM (Read Only Memory)); and a communication unit. When the processor loads a program stored in the recording medium (for example, the ROM) to the RAM, and executes the program, the control computer 18 functions as an apparatus comprising an output section 10, a control section 12, an arithmetic section 13 and a detection section 14. The control computer 18 may also include an auxiliary memory such as a semiconductor memory (EEPROM or flash memory) or a hard disk.

The control section 12 controls the timing for applying the voltage and the value of the voltage to be applied. The power supply device 11 includes a battery 16, and supplies electricity to the control computer 18 and the potentiostat 19 for operation. It is also possible to dispose the power supply device 11 outside the housing. The potentiostat 19 is a device which maintains the potential of the working electrode constant with respect to the potential of the reference electrode. The potentiostat 19, which is controlled by the control section 12, applies a predetermined amount of voltage between the counter electrode and the working electrode of the glucose sensor 17 using terminals CR and W; measures the response current of the working electrode which can be obtained at the terminal W; and sends the result of the measurement to the detection section 14.

The arithmetic section 13 calculates the concentration of the substance to be measured based on the value of the detected current, and stores the calculated result. The output section 10 carries out data communication between the output section 10 and the display section unit 15, and sends the calculated result of the concentration of the substance to be measured, provided by the arithmetic section 13, to the display section unit 15. The display section unit 15 is capable of displaying, for example, the calculated result of the glucose concentration which is received from the measuring apparatus B, on a display screen in a predetermined format.

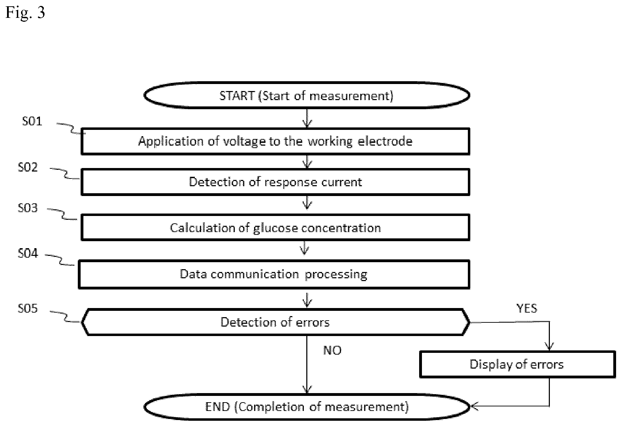

FIG. 3 is a flow chart showing an example of the processing sequence of the glucose concentration measurement carried out by the control computer 18. When the CPU (control section 12) of the control computer 18 receives an instruction to start the measurement of the glucose concentration, the control section 12 controls the potentiostat 19 to apply a predetermined amount of voltage to the working electrode, and starts measuring the response current from the working electrode (Step S01). Further, the detection of the installation of a sensor to the measuring apparatus may be used as the instruction to start the measurement of the concentration.

Next, the potentiostat 19 measures the response current generated by the application of voltage, specifically, the charge transfer limiting current based on the transfer to the electrode of electrons derived from the substance to be measured (glucose, in this embodiment) in the sample, preferably, the steady-state current observed after the occurrence of the transient current due to the charging of an electric double layer, for example, the steady-state current observed 1 to 20 seconds after the application of voltage. Then the potentiostat 19 sends the measured current to the detection section 14 (Step S02).

The arithmetic section 13 carries out arithmetic processing based on the measured current value, and calculates the glucose concentration (Step S03). For example, the formulae for calculating the glucose concentration or the data of the calibration curve of the glucose concentration, which correspond to the glucose dehydrogenase disposed on the electrode, are preinstalled to the arithmetic section 13 in the control computer 3, and the arithmetic section 13 calculates the glucose concentration utilizing these calculation formulae or the calibration curve.

The output section 10 sends the calculated result of the glucose concentration to the display section unit 15, through a communication link provided between the output section 10 and the display section unit 15 (Step S04). Thereafter, the control section 12 determines if there are any measurement errors detected (Step S05); completes the measurement if there is no error; and displays the glucose concentration on the display section. If there are any errors, a notification of error is displayed, and then the flow sequence shown in FIG. 3 is completed. Further, the calculated result can be stored in the arithmetic section 13, so that the stored result can be reloaded afterwards to be displayed on the display section for confirmation. Although the detection of measurement errors by the control section 12 (Step S05) is carried out after the calculated result is sent to the display section unit 15 (Step S04) in this embodiment, it is also possible to carry out these steps in different orders.

### EXAMPLES

Examples of the enzyme electrode will now be described. Example 1 does not form part of the invention.

### (Test 1)

### (Preparation of reagent solution)

Two types of reagent solutions according to Example 1 and Comparative Example 1 as described below were prepared.

### (Example 1)

### Ketjenblack (Mitsubishi Carbon Black): 1.20%

Electrically conductive polymer: sulfonated polyaniline aqueous solution (trade name: aquaPASS-01x; manufactured by Mitsubishi Rayon Co., Ltd.): 0.40% Oxazoline group-containing polymer, Epocros WS-700 (manufactured by NIPPON SHOKUBAI Co., Ltd.): 6.0%
Enzyme (Cy-GDH): 4.5 mg/mL
Sugar (sucrose): 0.50%
Phosphate buffer solution (pH 5.8): 10 mM
Note that, "%" represents the percent by weight concentration of the reagent contained in the reagent solution.

### (Comparative Example 1)

### Ketjenblack (Mitsubishi Carbon Black): 1.20%

Electrically conductive polymer: sulfonated polyaniline aqueous solution (trade name: aquaPASS-01x; manufactured by Mitsubishi Rayon Co., Ltd.): 0.40% Oxazoline group-containing polymer, Epocros WS-700 (manufactured by NIPPON SHOKUBAI Co., Ltd.): 6.0%
Enzyme (Cy-GDH): 4.5 mg/mL
Phosphate buffer solution (pH 5.8): 10 mM
Note that, "%" represents the percent by weight concentration of the reagent contained in the reagent solution. As can be seen from the above, the reagent solution according to Comparative Example 1 has the same composition as the reagent solution according to the Example 1, except that it contains no sugar.

### (Preparation of enzyme electrode (sample))

Next, a plurality of insulating base plates each having on one surface thereof an electrode (electrode layer) formed by gold vapor deposition (base materials) were prepared. The reagent solution according to Example 1 or Comparative Example 1 was dispensed on each of the insulating base plates, and the resulting base materials were dried by allowing them to stand for 30 minutes in a low humidity drying furnace. The reagents were thus allowed to solidify on respective electrodes to form detection layers, to obtain enzyme electrodes (samples) according to Example 1 and enzyme electrodes (samples) according to Comparative Example 1, each having a detection layer formed on one surface thereof.

### (Measurement of glucose concentration)

Next, the response current value was measured for a human whole blood sample in which the concentration of glucose was adjusted to 343 mg/dL (sample: S); or a human whole blood sample in which the concentration of glucose was adjusted to 0 mg/dL (blank: B), using each of the enzyme electrodes (working electrodes). The glucose measurement was carried out using a reference electrode/counter electrode (both made of carbon) and a reference electrode (silver/silver chloride electrode) formed on the electrode base plate, with a voltage of + 0.2 V applied to the working electrode (vs. Ag / AgCl).

### (Evaluation of measurement results)

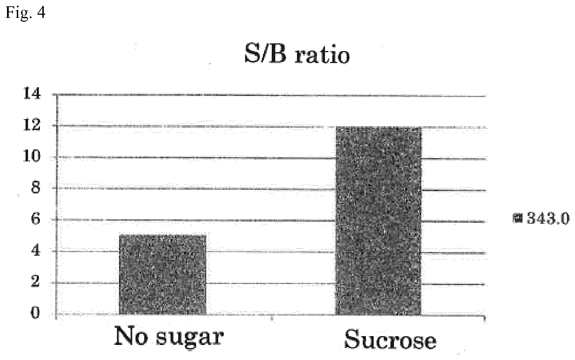

FIG. 4 is a graph showing the S/B ratio of the current values obtained by using the enzyme electrodes according to Comparative Example 1 and Example 1. Based on the test results shown in FIG. 4, it can be seen that the detection sensitivity is improved when the enzyme electrodes according to Example 1 were used, in which each of the detection layers contained a sugar, compared to the cases where the enzyme electrodes according to Comparative Example 1 were used, in which each of the detection layers contained no sugar.

### Comparative Example 2

Enzyme electrodes each comprising on one surface thereof a detection layer formed using a reagent solution having the following composition were prepared, in the same manner as described above.

### Ketjenblack (Mitsubishi Carbon Black): 1.20%

Electrically conductive polymer: sulfonated polyaniline aqueous solution (trade name: aquaPASS-01x; manufactured by Mitsubishi Rayon Co., Ltd.): 0.40% Enzyme (Cy-GDH): 4.5 mg/mL
Sugar(sucrose): 0.50%
Phosphate buffer solution (pH 5.8): 10 mM
Note that, "%" represents the percent by weight concentration of the reagent contained in the reagent solution. As can be seen from the above, the reagent solution according to Comparative Example 2 has the same composition as the reagent solution according to Example 1, except that it contains no crosslinking agent.

The response current value was measured for an aqueous solution containing 343 mg/dl of glucose (sample: S) or for water (blank: B), using the enzyme electrodes according to Comparative Example 2. As a result, even in the case where the aqueous solution of glucose was used, the current was barely detectable, and it was impossible to measure the glucose concentration.

### (Test 2)

Enzyme electrodes each comprising a detection layer having the same composition as those prepared in Example 1 except for containing one of various types of sugar instead of sucrose were prepared. Then, using the thus prepared enzyme electrodes, the response current value was measured for a human whole blood sample whose glucose concentration was adjusted to 256 mg/dl or to 512 mg/dl (sample: S); or a human whole blood sample whose glucose concentration was adjusted to 0 mg/dL (blank: B), and the S/B ratio was obtained for each of the samples, in the same manner as in Example 1. The enzyme electrodes in which the detection layer contains a sugar which is sucrose, maltotriose, maltohexaose, fructose, xylitol or mannose do not form part of the invention.

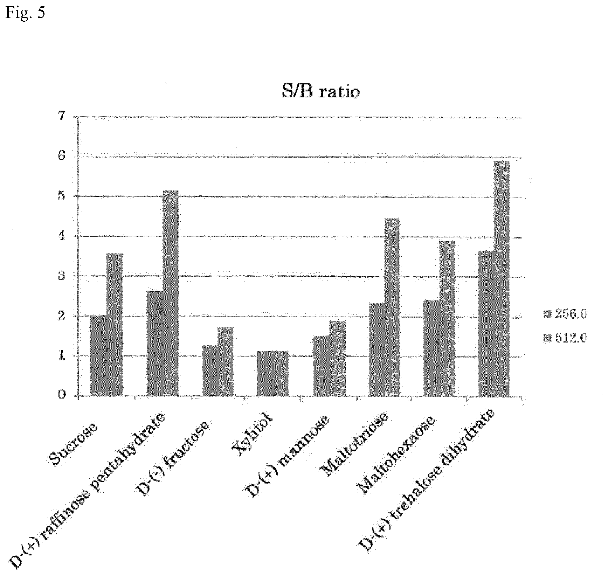

FIG. 5 is a graph showing the results of the test 2, specifically, a graph of the S/B ratio of the current obtained by using the enzyme electrodes each comprising a detection layer containing one of various types of sugar. According to the test results shown in FIG. 5, the S/B ratio was low when the enzyme electrodes each comprising a detection layer containing xylitol, mannose or fructose were used, and the concentration dependence was barely observed. However, when the enzyme electrodes each comprising a detection layer containing trehalose, sucrose, maltotriose, raffinose or maltohexaose were used, the S/B ratio was improved, and the concentration dependence for glucose was observed. These results have revealed that the incorporation of a sugar which is trehalose or raffinose in the detection layer of the enzyme electrode allows for an improvement in the detection sensitivity of the enzyme electrode, and for a quantitative measurement of the substance to be measured.

### DESCRIPTION OF SYMBOLS

- A: enzyme electrode
- 1: electrode
- 2: detection layer
- 3: insulating base plate
- 4: enzyme
- 5: electrically conductive polymer
- 6: sugar
- 7: crosslinking agent (main chain)
- 7': crosslinking agent (side chain)
- B: measuring apparatus
- 10: output section
- 11: power supply device
- 12: control section
- 13: arithmetic section
- 14: detection section
- 15: display section unit
- 16: battery
- 17: glucose sensor
- 18: control computer
- 19: potentiostat
- 20: base plate
- CR, W: terminals

## Claims

1. An enzyme electrode (A), comprising:
an electrode (1); and
a detection layer (2) which is in contact with the electrode, and which comprises an enzyme (4), a crosslinking agent (7), an electrically conductive polymer (5), and electrically conductive particles,
wherein the detection layer (2) does not contain an electron transfer mediator which is an oxidation-reduction substance;
**characterized in that** the crosslinking agent (7) is an oxazoline group-containing compound, and the detection layer (2) further comprises a sugar (6) which is trehalose or raffinose.

2. The enzyme electrode (A) according to claim 1, wherein the molecules of the enzyme (4) are crosslinked by the crosslinking agent (7).

3. The enzyme electrode (A) according to claim 1 or claim 2, wherein the enzyme (4) is an oxidoreductase.

4. The enzyme electrode according to claim 3, wherein the enzyme is a saccharide oxidoreductase selected from glucose oxidase, galactose oxidase, glucose dehydrogenase, fructose dehydrogenase and sorbitol dehydrogenase.

5. The enzyme electrode according to any one of claims 1 to 4, wherein the sugar (6) is a sugar which does not serve as a substrate for the enzyme (4).

6. A biosensor comprising the enzyme electrode according to any one of claims 1 to 5.

7. A biosensor according to claim 6 which further comprises a counter electrode.

8. Use of a biosensor according to claim 6 or claim 7 as a glucose or cholesterol sensor.

9. Use of a biosensor according to claim 8 to measure the concentration of glucose in a blood sample.

10. An apparatus (B) for measuring the concentration of a substance in a sample comprising:
the biosensor according to claim 6 or 7;
a control section (12) configured to control the application of voltage to the biosensor;
a detection section (14) configured to detect a charge transfer limiting current based on the transfer to the electrode of electrons derived from said substance, wherein the charge transfer limiting current is generated by the application of voltage to the biosensor;
an arithmetic section (13) configured to calculate the concentration of the substance based on the value of the charge transfer limiting current; and
an output section (10) configured to output the calculated concentration of the substance.

11. A method for producing an enzyme electrode according to any one of claims 1 to 5 comprising forming a detection layer on an electrode by applying a mixed solution on said electrode, wherein the mixed solution comprises an enzyme, a crosslinking agent which is an oxazoline group-containing compound, an electrically conductive polymer, electrically conductive particles, and a sugar which is trehalose or raffinose, and wherein the detection layer does not contain an electron transfer mediator which is an oxidation-reduction substance.

## Patentansprüche

1. Enzymelektrode (A), umfassend:
eine Elektrode (1); und
eine Nachweisschicht (2), die mit der Elektrode in Kontakt steht und die ein Enzym (4), ein Vernetzungsmittel (7), ein elektrisch leitfähiges Polymer (5) und elektrisch leitfähige Teilchen umfasst,
wobei die Nachweisschicht (2) keinen Elektronentransfermediator enthält, der eine Oxidations-Reduktions-Substanz ist;
**dadurch gekennzeichnet, dass** das Vernetzungsmittel (7) eine Oxazolingruppen-haltige Verbindung ist und die Nachweisschicht (2) weiter einen Zucker (6) umfasst, der Trehalose oder Raffinose ist.

2. Enzymelektrode (A) nach Anspruch 1, wobei die Moleküle des Enzyms (4) durch das Vernetzungsmittel (7) vernetzt sind.

3. Enzymelektrode (A) nach Anspruch 1 oder Anspruch 2, wobei das Enzym (4) eine Oxidoreduktase ist.

4. Enzymelektrode nach Anspruch 3, wobei das Enzym eine Saccharid-Oxidoreduktase ist, ausgewählt aus Glucoseoxidase, Galactoseoxidase, Glucosedehydrogenase, Fructosedehydrogenase und Sorbitdehydrogenase.

5. Enzymelektrode nach einem der Ansprüche 1 bis 4, wobei der Zucker (6) ein Zucker ist, der nicht als Substrat für das Enzym (4) dient.

6. Biosensor, der die Enzymelektrode nach einem der Ansprüche 1 bis 5 umfasst.

7. Biosensor nach Anspruch 6, der weiter eine Gegenelektrode umfasst.

8. Verwendung eines Biosensors nach Anspruch 6 oder Anspruch 7 als Glucose- oder Cholesterinsensor.

9. Verwendung eines Biosensors nach Anspruch 8, um die Glucosekonzentration in einer Blutprobe zu messen.

10. Einrichtung (B) zum Messen der Konzentration einer Substanz in einer Probe, umfassend:
den Biosensor nach Anspruch 6 oder 7;
einen Steuerabschnitt (12), der so konfiguriert ist, dass er das Anlegen von Spannung an den Biosensor steuert;
einen Nachweisabschnitt (14), der so konfiguriert ist, dass er einen ladungstransferbegrenzenden Strom auf Grundlage des Transfers von Elektronen, die aus der Substanz stammen, zur Elektrode nachweist, wobei der ladungstransferbegrenzende Strom durch das Anlegen von Spannung an den Biosensor erzeugt wird;
einen arithmetischen Abschnitt (13), der so konfiguriert ist, dass er die Konzentration der Substanz auf Grundlage des Wertes des ladungstransferbegrenzenden Stroms berechnet; und
einen Ausgabeabschnitt (10), der so konfiguriert ist, dass er die berechnete Konzentration der Substanz ausgibt.

11. Verfahren zur Herstellung einer Enzymelektrode nach einem der Ansprüche 1 bis 5, das das Bilden einer Nachweisschicht auf einer Elektrode durch Aufbringen einer gemischten Lösung auf die Elektrode umfasst, wobei die gemischte Lösung ein Enzym, ein Vernetzungsmittel, das eine Oxazolingruppen-haltige Verbindung ist, ein elektrisch leitfähiges Polymer, elektrisch leitfähige Teilchen und einen Zucker, der Trehalose oder Raffinose ist, umfasst, und wobei die Nachweisschicht keinen Elektronentransfermediator enthält, der eine Oxidations-Reduktions-Substanz ist.

## Revendications

1. Électrode à enzyme (A), comprenant :
une électrode (1) ; et
une couche de détection (2) qui est en contact avec l'électrode et qui comprend une enzyme (4), un agent de réticulation (7), un polymère électriquement conducteur (5) et des particules électriquement conductrices,
dans laquelle la couche de détection (2) ne contient pas de médiateur de transfert d'électrons qui est une substance d'oxydo-réduction ;
**caractérisée en ce que** l'agent de réticulation (7) est un composé contenant un groupe oxazoline, et la couche de détection (2) comprend en outre un sucre (6) qui est du tréhalose ou du raffinose.

2. Électrode à enzyme (A) selon la revendication 1, dans laquelle les molécules de l'enzyme (4) sont réticulées par l'agent de réticulation (7).

3. Électrode à enzyme (A) selon la revendication 1 ou la revendication 2, dans laquelle l'enzyme (4) est une oxydoréductase.

4. Électrode à enzyme selon la revendication 3, dans laquelle l'enzyme est une saccharide oxydoréductase choisi parmi la glucose oxydase, la galactose oxydase, la glucose déshydrogénase, la fructose déshydrogénase et la sorbitol déshydrogénase.

5. Électrode à enzyme selon l'une quelconque des revendications 1 à 4, dans laquelle le sucre (6) est un sucre qui ne sert pas de substrat pour l'enzyme (4).

6. Biocapteur comprenant l'électrode à enzyme selon l'une quelconque des revendications 1 à 5.

7. Biocapteur selon la revendication 6 qui comprend en outre une contre-électrode.

8. Utilisation d'un biocapteur selon la revendication 6 ou la revendication 7 comme capteur de glucose ou de cholestérol.

9. Utilisation d'un biocapteur selon la revendication 8 pour mesurer la concentration de glucose dans un échantillon de sang.

10. Appareil (B) de mesure de la concentration d'une substance dans un échantillon comprenant :
le biocapteur selon la revendication 6 ou 7 ;
une section de commande (12) configurée pour commander l'application de tension au biocapteur ;
une section de détection (14) configurée pour détecter un courant de limitation de transfert de charge sur la base du transfert à l'électrode d'électrons dérivés de ladite substance, dans lequel le courant de limitation de transfert de charge est généré par l'application de tension au biocapteur ;
une section arithmétique (13) configurée pour calculer la concentration de la substance sur la base de la valeur du courant de limitation de transfert de charge ; et
une section de sortie (10) configurée pour délivrer en sortie la concentration calculée de la substance.

11. Procédé de production d'une électrode à enzyme selon l'une quelconque des revendications 1 à 5 comprenant la formation d'une couche de détection sur une électrode en appliquant une solution mixte sur ladite électrode, dans lequel la solution mixte comprend une enzyme, un agent de réticulation qui est un composé contenant un groupe oxazoline, un polymère électriquement conducteur, des particules électriquement conductrices et un sucre qui est du tréhalose ou du raffinose, et dans lequel la couche de détection ne contient pas de médiateur de transfert d'électrons qui est une substance d'oxydo-réduction.
